# EUROPÄISCHE PATENTSCHRIFT

(11) **EP 2 361 047 B1**
(45) Veröffentlichungstag und Bekanntmachung des Hinweises auf die Patenterteilung: **17.12.2014**
(21) Anmeldenummer: 09744021.8
(22) Anmeldetag: 19.10.2009
(51) Int. Cl.: A61B 17/80, A61B 17/15, A61B 17/17

(54) **VORRICHTUNG ZUM VERKÜRZEN EINES LÄNGLICHEN KNOCHENS**
LONG BONE SHORTENING DEVICE
DISPOSITIF POUR RACCOURCIR UN OS LONGUE

(30) Priorität: 27.11.2008 AT 18512008
(43) Veröffentlichungstag der Anmeldung: 31.08.2011
(73) Patentinhaber: I.T.S. GmbH, 8301 Lassnitzhöhe (AT)
(72) Erfinder: BERNSTEINER, René, 8043 Graz (AT)
(74) Vertreter: Wirnsberger, Gernot
(86) Internationale Anmeldenummer: PCT/AT2009/000405
(87) Internationale Veröffentlichungsnummer: WO 2010/060124

(56) Entgegenhaltungen:
- US-A- 4 929 247
- US-A1- 2007 270 850
- US-A1- 2007 276 383

## Beschreibung

Die Erfindung betrifft eine Vorrichtung zum Verkürzen eines länglichen Knochens, insbesondere einer Ulna, umfassend ein Halteelement und eine am Knochen anzubringende Knochenplatte mit einem Loch für ein Befestigungselement zum Fixieren der Knochenplatte am Knochen und einem längs dazu versetzten Langloch für das Halteelement, das im Knochen befestigbar ist, sodass Knochenteile nach Durchtrennen des Knochens und Entnahme eines Knochenstücks im Bereich zwischen dem Loch und dem Langloch durch Verschieben der Knochenplatte relativ zum Halteelement zusammenführbar sind.

Eine eingeschränkte Bewegungsfreiheit eines Handgelenks eines Menschen kann durch eine zu starke Krümmung einer Ulna bedingt sein. Eine Möglichkeit, dies zu korrigieren und dadurch eine Bewegungsfreiheit des Handgelenks zu erhöhen, besteht darin, die Ulna zu verkürzen. Dabei wird so vorgegangen, dass die Ulna an zwei Stellen entlang paralleler Ebenen durchtrennt wird, nach Durchtrennen ein Knochenstück der Ulna entnommen wird und danach die Knochenteile im Bereich der parallelen Ebenen zusammengeführt werden. Die Knochenteile wachsen anschließend im Bereich der zusammengeführten Ebenen zusammen, sodass nach dem Zusammenwachsen der Knochenteile eine verkürzte und weniger stark gekrümmte Ulna vorliegt.

Ein Verkürzen einer Ulna muss mit hoher Präzision erfolgen, sodass lediglich deren Krümmung korrigiert wird, nicht jedoch durch den vorgenommenen Eingriff Fehlstellungen hervorgerufen werden, z. B. eine verdrehte Stellung der temporär getrennt vorliegenden Knochenteile aufgrund Verdrehens der Knochteile beim Zusammenführen und während des nachfolgenden Zusammenwachsens derselben.

Gemäß dem Stand der Technik sind bereits verschiedene Vorrichtungen zum Verkürzen einer Ulna vorgeschlagen worden. Aus der Druckschrift US 2005/0277941 A1 ist eine Knochenplatte bekannt geworden, die nach dem Verkürzen der Ulna am Knochen befestigt bleibt und mit zusätzlichen Einrichtungen ausgestattet ist, die während einer Entnahme eines Knochenstücks mit der Knochenplatte zusammenwirken und zum Halten von getrennten Knochenteilen, welche nach der Entnahme eines Knochenstücks vorliegen, und deren Zusammenführen dienen. Diese Einrichtungen umfassen eine Führungseinrichtung für eine Säge zum Erzeugen paralleler Schnitte im Knochen bzw. zum Durchtrennen desselben. Des Weiteren sind Halteelemente in der Form von Nägeln vorgesehen, die in Langlöchern der Knochenplatte sitzen und im Knochen befestigt werden. Die Knochenplatte ist zumindest an einer weiteren Position mit einer Schraube am Knochen schubfest befestigt. Nach Durchtrennen des Knochens und Entnahme eines Knochenstücks wird eine Zange an der Knochenplatte im befestigten Bereich angesetzt und die getrennten Knochenteile zusammengeführt, wobei die Zange in einer weiteren Position an den vorgesehenen Nägeln angreifen kann, die als Halt dienen, sodass die Knochenplatte mit einem Knochenteil relativ zu den in den vorgesehenen Langlöchern sitzenden Nägeln und dem anderen Knochteil bewegt wird. Wenngleich eine Vorrichtung dieser Art ein Zusammenführen von Knochenteilen erlaubt, so ist doch nachteilig, dass eine Verkürzung einer Ulna mit dieser Vorrichtung von einer einzelnen Person kaum oder nicht durchgeführt werden kann. Die Knochenplatte muss nämlich nach Zusammenführen der Knochenteile an einem Knochenteil noch fixiert werden. Gleichzeitig ist es allerdings erforderlich, die Knochenteile mit der Zange in Kontakt zu halten, weshalb sich die Handhabung bei einer Verkürzung einer Ulna bzw. einer Operation schwierig gestaltet. Darüber hinaus ist das händische Zusammenführen mithilfe der Zange eine potenzielle Fehlerquelle, da keine exakte Kontrolle über das Zusammenführen der Knochenteile gegeben ist.

Aus der Druckschrift US 4,929,247 ist eine Vorrichtung bekannt geworden, die ebenfalls beim Verkürzen einer Ulna eingesetzt werden kann. Diese Vorrichtung umfasst eine Knochenplatte mit mehreren Löchern zum Befestigen der Knochenplatte am Knochen sowie einem Langloch. Daneben umfasst die Vorrichtung zwei Blöcke, die beidseits eines zu entnehmenden Knochenstücks mit langen Haltebolzen am Knochen angebracht werden, wobei ein Haltebolzen durch das Langloch geführt ist. Schließlich sind die zwei auf der Knochenplatte senkrecht in die Höhe ragend angeordneten Blöcke mit zwei parallel zur Knochenplatte bzw. waagrecht verlaufenden Schrauben verbunden, durch welche die Blöcke relativ zueinander bewegt werden können. Ist im Bereich zwischen den Blöcken ein Knochenstück entnommen, so werden die Stellschrauben betätigt und die Knochenteile dadurch zueinander bewegt. Im Anschluss wird die Knochenplatte am Knochen fixiert und die Blöcke von der Knochenplatte abgenommen, indem die Haltebolzen gelöst werden. Bei dieser Vorrichtung ist allerdings nachteilig, dass diese wegen der zwei erforderlichen Blöcke einen großen Platzbedarf aufweist, was bei einer Operation zu Nachteilen in der Handhabung der Vorrichtung führen kann, da man bei einer Operation in der Regel nur einen kurzen Schnitt anbringen will und daher ein zur Verfügung stehender Platz gering ist.

Aus der Druckschrift US 5,042,983 ist eine Schneideinrichtung zur Entnahme eines Knochenstücks einer Ulna bekannt geworden. Diese Vorrichtung wird in Verbindung mit einer Vorrichtung gemäß der zuvor genannten Druckschrift US 4,929,247 eingesetzt. Die Schneideinrichtung ist auf einer Trägerplatte positioniert, die zur Knochenplatte gemäß Druckschrift US 4,929,247 korrespondiert. In einem ersten Schritt wird die Schneideinrichtung mit der entsprechenden Trägerplatte am Knochen in vorgegebenen Positionen befestigt. Nach Durchtrennen des Knochens und Entnahme eines Knochenstücks wird die Schneideinrichtung bzw. die Trägerplatte abgenommen und die Knochenplatte gemäß Druckschrift US 4,929,247 aufgesetzt, um die Knochenteile zusammenzuführen. Bei dieser Vorgehensweise ist allerdings nachteilig, dass neben den zuvor dargestellten Nachteilen die Knochenplatte lagegenau in jenen Löchern befestigt werden muss, in der auch die Trägerplatte der Schneideinrichtung zuvor positioniert war, was eine Unsicherheit in Bezug auf die Lage der voneinander getrennt vorliegenden Knochenteile beim Zusammenführen derselben mit sich bringt.

Eine Entnahme eines Knochenstücks einer Ulna und eine nachträgliche Anordnung einer Vorrichtung samt Knochenplatte sowie ein Zusammenführen von Knochenteilen in zwei getrennten Schritten bzw. mit zwei gesonderten Vorrichtungen ist auch aus der Druckschrift US 2007/0276383 A1 bekannt geworden.

Aufgabe der Erfindung ist es, eine Vorrichtung der eingangs genannten Art anzugeben, die mit geringem Platzbedarf baut und ein hochgenaues Zusammenführen der Knochenteile erlaubt.

Diese Aufgabe wird bei einer Vorrichtung der eingangs genannten Art gelöst, wenn auf der Knochenplatte im Bereich des Langlochs ein Block mit einer in Draufsicht länglichen und über dem Langloch liegenden Öffnung für das Halteelement angeordnet ist, wobei in der Öffnung eine Einheit des Blockes angeordnet ist, an welcher das Halteelement in Verschieberichtung zur Anlage kommt und relativ zu welcher der übrige Teil des Blockes mithilfe eines vorgesehenen Stellelementes verschiebbar ist.

Bei einer erfindungsgemäßen Vorrichtung ist von Vorteil, dass diese nur einen Block aufweist, der mit einem Halteelement zusammenwirkt, sodass eine kompakte Bauweise gegeben ist. Bei Verwendung der Vorrichtung liegt das Halteelement in Verschieberichtung der Knochenplatte gesehen an der in der Öffnung positionierten Einheit an, sodass sich die Einheit nicht gegen die Verschieberichtung bewegen kann. Daher ist lediglich eine Bewegung des übrigen Teils des Blockes in Verschieberichtung möglich, was mit dem vorgesehenen Stellelement bewirkt werden kann. Darüber hinaus ist auch ein hochgenaues Zusammenführen von Knochenteilen nach Entnahme eines Knochenstücks möglich, da der Block bereits auf der Knochenplatte angeordnet ist und unmittelbar nach Zusammenführen der Knochenteile ein schubfestes Befestigen der Knochenplatte auch am zweiten Knochenteil erfolgen kann. Nach Befestigen der Knochenplatte kann dann der Block abgenommen werden.

Die erfindungsgemäße Vorrichtung kann grundsätzlich zum Verkürzen beliebiger Knochen bei Menschen oder Tieren eingesetzt werden. Bevorzugt kommt die erfindungsgemäße Vorrichtung jedoch beim Verkürzen einer Ulna zum Einsatz. Für diese Zwecke ist die Knochenplatte entsprechend einem bereichsweisen Verlauf der Ulna länglich ausgebildet.

Der vorgesehene Block kann unmittelbar auf der Knochenplatte angeordnet sein. Besonders bevorzugt ist es jedoch, dass der Block bodenseitig mit Fortsätzen ausgebildet ist, die eine weitere Platte definieren, die zumindest teilweise etwa mit einer gleichen Breite wie die Knochenplatte, jedoch kürzer ausgebildet ist. Durch die vorgesehenen Fortsätze, die sich wie erwähnt bodenseitig befinden, wird ein Bereich zum Befestigen des Blockes auf der Knochenplatte geschaffen. Da die Fortsätze bodenseitig angeordnet und flach ausgebildet sind, ist dadurch kein Nachteil in Bezug auf einen Platzbedarf gegeben. Der Block ist dabei zweckmäßigerweise etwa mittig auf der Knochenplatte angeordnet, was erfordert, dass auch das Langloch der Knochenplatte etwa mittig derselben angeordnet ist. Dies bringt den Vorteil mit sich, dass der Block, der bei einer Operation den größten Platzbedarf erfordert, etwa in der Mitte eines Schnittes z. B. entlang eines Unterarms liegt, wo die Haut am weitesten aufgespreizt werden kann und somit der meiste Platz zum Anbringen der Vorrichtung zur Verfügung steht. Grundsätzlich kann der Block auch ungefähr endseitig der Knochenplatte angeordnet sein, wenngleich sich in diesem Fall eine Operation etwas schwieriger gestaltet, da der Block dann im Bereich eines Endes eines Schnittes zusammen mit der Knochenplatte am Knochen befestigt werden muss. In jedem Fall ist es zweckmäßig, dass die Knochenplatte ein weiteres Langloch und die weitere Platte ein Langloch aufweisen, welche Langlöcher übereinander angeordnet sind und sich an einem dem Loch gegenüberliegenden Ende der Knochenplatte befinden. Es können dann zwei Halteelemente eingesetzt werden, wobei ein Halteelement mit dem Block in Eingriff steht und das weitere Halteelement einer geradlinigen Führung der Knochenplatte beim Zusammenführen der Knochenteile dient.

Damit die Knochenplatte möglichst ohne auch nur geringfügiges Verdrehen eines der Knochenteile relativ zueinander bewegt werden kann, ist eine erfindungsgemäße Vorrichtung zweckmäßigerweise so ausgebildet, dass das Halteelement und ein weiteres Halteelement vorgesehen sind, wobei das Halteelement in das Langloch der Knochenplatte und die Öffnung des Blockes und das weitere Halteelement in das weitere Langloch der Knochenplatte und in das Langloch der weiteren Platte eingreifen und länglich mit einem zylindrischen Teil und einem ein Gewinde aufweisenden Teil gebildet sind, wobei der zylindrische Teil des Halteelementes im Block an der Einheit und der zylindrische Teil des weiteren Haltelementes auf der weiteren Platte anliegt. Diesbezüglich erweist es sich als zweckmäßig, dass die Langlöcher der Knochenplatte und ein freier Bereich der Öffnung, in den das Halteelement eingeführt ist, und das Langloch der weiteren Platte mit etwa gleichen Abmessungen ausgebildet sind.

Die weitere Platte ist in der Regel lösbar auf der Knochenplatte angebracht, sodass die weitere Platte zusammen mit dem Block von der Knochenplatte getrennt werden kann, sobald die Knochenteile aneinanderliegen und durch die Knochenplatte jeweils fixiert sind.

Besonders zweckmäßig ist es, dass an der weiteren Platte eine Führungseinrichtung für ein Schneidgerät lösbar befestigt ist, z. B. seitlich der Führungseinrichtung. Dies ermöglicht es, einen Knochen möglichst präzise zu durchtrennen. Um Schnitte entlang eines Knochens variabel setzen zu können, kann dabei vorgesehen sein, dass die Führungseinrichtung relativ zur weiteren Platte variabel fixierbar ist. Es ist dann ausreichend, dass die Führungseinrichtung lediglich einen Führungsschlitz aufweist. Wenn zudem eine Skala vorgesehen ist, an welcher eine Position der Führungseinrichtung relativ zur weiteren Platte ablesbar ist, so kann ein Knochenstück mit einer gewünschten bzw. vorgegebenen Länge entnommen werden. Dies war bislang gemäß dem Stand der Technik nicht oder nur in Einzelfällen möglich, da nur Führungseinrichtungen mit mehreren parallelen Führungsschlitzen vorgegebenen konstanten Abstands vorgesehen waren. Dies erlaubte lediglich eine Entnahme von Knochenstücken, die einem ganzzahligen Vielfachen des Abstands zwischen zwei Führungsschlitzen entsprachen.

Die Führungseinrichtung ist vorzugsweise lösbar auf der weiteren Platte befestigt, sodass die Führungseinrichtung nach Durchtrennen des Knochens, aber noch vor Zusammenführen der Knochenteile abgenommen werden kann und damit beim Zusammenführen der Knochenteile nicht stört.

Um ein hochgenaues Zusammenführen der Knochenteile zu ermöglichen bzw. einen Anpressdruck der Knochenteile vor Fixierung der Knochenplatte zu kontrollieren, ist der übrige Teil des Blockes in vorteilhafter Weise durch das Stellelement kontinuierlich verschiebbar.

Ebenfalls in Bezug auf ein exaktes Zusammenführen der Knochenteile kann vorgesehen sein, dass die Einheit das Halteelement teilweise umfasst. Durch diese Maßnahme ist eine Positionsänderung des Halteelementes relativ zur Einheit verhindert oder zumindest weitgehend ausgeschlossen.

Das Stellelement kann als Stellschraube ausgebildet sein, welche im oder am übrigen Teil des Blockes angeordnet ist und mit der Einheit in Verbindung steht. Dabei ist es zweckmäßig, dass die Stellschraube etwa parallel zur Knochenplatte in Verschieberichtung verläuft.

Von Vorteil ist es des Weiteren, dass die Einheit in die Öffnung einsetzbar ist und der Block in Verschieberichtung verlaufende seitliche Ausnehmungen aufweist, durch welche ein mit dem Stellelement in Verbindung stehendes und die Einheit vertikal in Position haltendes Haltemittel geführt ist. Dies trägt zu einem ruhigen Verschieben des übrigen Teils des Blockes relativ zur Einheit bei, da das Haltemittel im Zusammenspiel mit den Ausnehmungen der Führung des übrigen Teils des Blockes dient.

Weitere Merkmale, Vorteile und Wirkungen der Erfindung ergeben sich aus dem nachfolgend dargestellten Ausführungsbeispiel sowie den Zeichnungen. In den Zeichnungen zeigen:
Fig. 1 eine erfindungsgemäße Vorrichtung in perspektivischer Darstellung;
Fig. 2 eine erfindungsgemäße Vorrichtung in einer Seitenansicht;
Fig. 3 die in Fig. 2 dargestellte Vorrichtung in stirnseitiger Ansicht;
Fig. 4 die in Fig. 2 dargestellte Vorrichtung in Draufsicht;
Fig. 5 eine schematische Darstellung des Anordnens einer erfindungsgemäßen Vorrichtung auf einem Knochen;
Fig. 6 eine schematische Darstellung des Durchtrennens eines Knochens;
Fig. 7 eine schematische Darstellung ähnlich Fig. 6 nach Entnahme eines Knochenstücks;
Fig. 8 einen Knochen mit einer befestigten Knochenplatte.

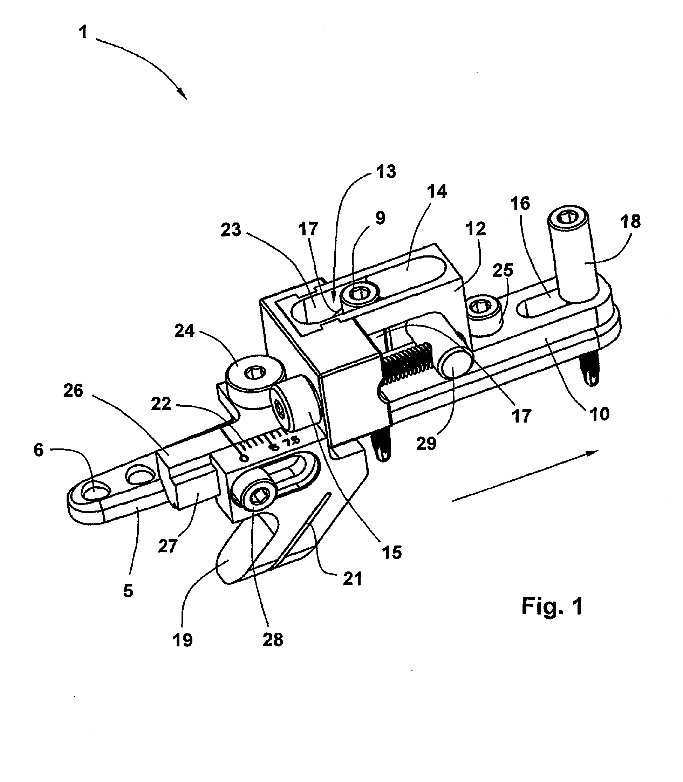

In Fig. 1 bis 4 ist eine erfindungsgemäße Vorrichtung 1 in verschiedenen Ansichten dargestellt. Die Vorrichtung 1 umfasst eine Knochenplatte 5, die ein endseitiges Loch 6 aufweist, das in Draufsicht etwa rund ausgebildet ist. Daneben weist die Knochenplatte 5 ein Langloch 7 sowie ein weiteres Langloch 11 auf, deren Positionen bzw. Lagen noch später erläutert werden. Die Knochenplatte 5 ist länglich ausgebildet und besteht in der Regel aus einem Metall wie Titan.

Auf der Knochenplatte 5 ist ein Block 12 angeordnet. Der Block 12 ist bodenseitig mit zwei sich entlang der Knochenplatte 5 erstreckenden Fortsätzen ausgebildet, die gemeinsam eine weitere Platte 10 definieren. Die weitere Platte 10 bzw. die Fortsätze des Blockes 12 sind mit Befestigungsmitteln 24, 25, z. B. Schrauben, lösbar auf der Knochenplatte 5 befestigt. An einem dem endseitig angeordneten Loch 6 der Knochenplatte 5 gegenüberliegenden Ende derselben erstreckt sich die weitere Platte 10 bzw. der entsprechende Fortsatz des Blockes 12 etwa bis zum Ende der Knochenplatte 5 und weist in diesem endseitigen Bereich ein Langloch 16 auf. Dieses Langloch 16 ist oberhalb des mit korrespondierender Dimensionierung ausgebildeten weiteren Langlochs 11 der Knochenplatte 5 positioniert. Das Langloch 11 ist in Fig. 1 bis 4 teilweise durch die weitere Platte 10 verdeckt und erst nach Abnahme der weiteren Platte 10 deutlich ersichtlich. In diesem Bereich ist die weitere Platte 10 mit einer Breite ausgebildet, die etwa jener der Knochenplatte 5 entspricht.

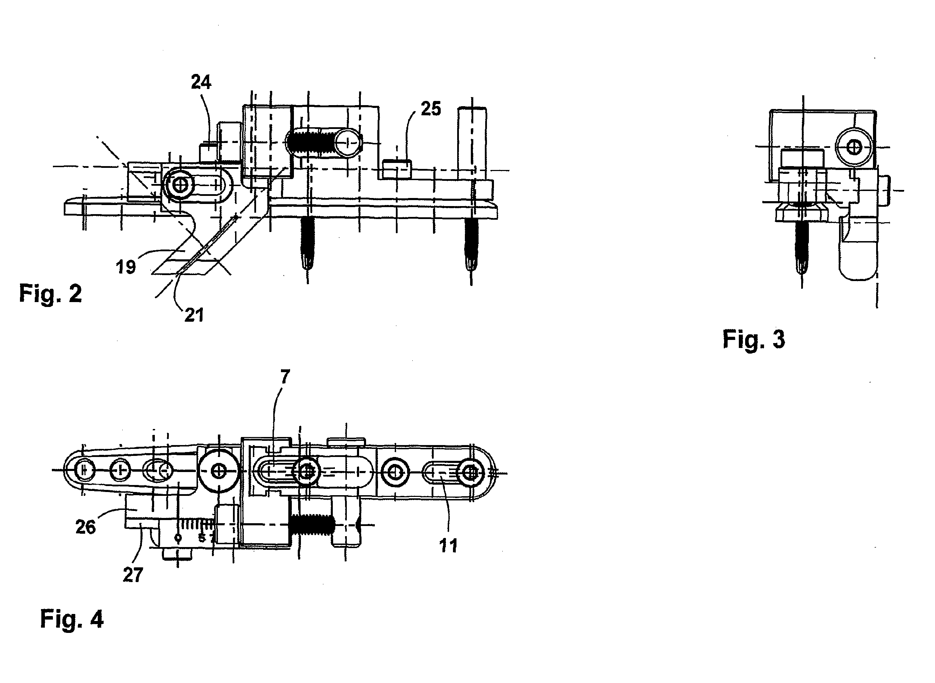

Der übrige Teil der weiteren Platte 10 bzw. des Fortsatzes des Blockes 12, der sich vom Block 12 zum endseitigen Loch 6 der Knochenplatte 5 erstreckt, ist in einem an den Block 12 anschließenden Bereich so ausgebildet, dass die weitere Platte 10 im Bereich des Befestigungsmittels 24 auf einer Seite etwa bündig mit der Knochenplatte 5 abschließt, auf der anderen Seite jedoch eine in Richtung des Lochs 6 der Knochenplatte 5 ragende Zunge 26 aufweist, was insbesondere aus Fig. 2 bis 4 ersichtlich ist. Die Zunge 26 weist außenseitig eine etwa parallel zur Knochenplatte 5 verlaufende Schiene 27 auf. Auf der Schiene 27 ist eine Führungseinrichtung 19 angeordnet. Die Führungseinrichtung 19 weist einen Führungsschlitz 21 auf, durch welchen beispielsweise eine Säge geführt werden kann, sodass die Säge während eines Durchtrennens einer Ulna die durch den Führungsschlitz 21 definierte Ebene nicht verlässt. Ein oberer Teil der Führungseinrichtung 19 umfasst die Schiene 27. Zu diesem Zweck ist die Führungseinrichtung 19 mit einer einem Profil der Schiene 27 entsprechenden Nut ausgebildet. Im oberen Teil der Führungseinrichtung 19 befindet sich des Weiteren eine Skala 22, anhand welcher eine Position der Führungseinrichtung 19 relativ zur weiteren Platte 10, gegenüber welcher die Führungseinrichtung 19 verschiebbar ist, ablesbar ist. Zu diesem Zweck ist auf der weiteren Platte 10 eine Markierung angebracht. Die Verschiebbarkeit der Führungseinrichtung 19 wird dadurch erreicht, dass die Führungseinrichtung 19 eine waagrecht verlaufende seitliche Öffnung aufweist, in der ein in die Schiene 27 eingreifendes Halteelement 28 angeordnet ist, das gelöst werden kann, sodass die Führungseinrichtung 19 entlang der Öffnung, je nach Bedarf bzw. Länge eines zu entnehmenden Knochenstücks, verschoben werden kann. Aufgrund der vorgesehenen Skala 22 kann eine Länge des zu entnehmenden Knochenstücks genau eingestellt werden. Dabei ist gleichzeitig der Vorteil gegeben, dass für zwei vorzunehmende Schnitte jeweils der gleiche Führungsschlitz 21 als Führungsmittel Anwendung findet, was darin resultiert, dass zwei im Wesentlichen parallele Trennebenen erhalten werden, was sich nach Entnahme eines Knochenstücks und Zusammenführen von Teilen einer Ulna bzw. beim Zusammenwachsen der Teile als günstig erweist. Wie beispielsweise aus Fig. 2 ersichtlich ist, ist der Führungsschlitz 21 in einem unteren Teil der Führungseinrichtung 19 angeordnet und verläuft etwa in einem Winkel von 45° zur Knochenplatte 5.

Der Block 12 ist etwa mittig auf der Knochenplatte 5 angeordnet. Wie erwähnt ist der Block 12 mit bodenseitigen Fortsätzen ausgebildet, die eine weitere Platte 10 definieren. Dies ist in Bezug auf eine einfache Handhabung der Vorrichtung 1 von Vorteil. Möglich ist es aber auch, dass der Block 12 und die weitere Platte 10 getrennt ausgeführt sind. In diesem Fall liegt der Block 12 auf der weiteren Platte 10 auf. Der Block 12 weist eine zentrale Öffnung 13 auf. Eine Länge dieser Öffnung 13 ist so bemessen, dass diese zumindest das darunterliegende Langloch 7 der Knochenplatte 5 überdeckt. Dadurch kann ein Halteelement 9 in der Öffnung 13 des Blockes 12 positioniert werden. Ein weiteres Halteelement 18 wird im Einsatz in den Langlöchern 7, 11 der Knochenplatte 5 bzw. der weiteren Platte 10 positioniert.

Wie aus Fig. 1 bis 4 ersichtlich ist, ist in der Öffnung 13 des Blockes 12 eine Einheit 14 angeordnet. Diese Einheit 14 füllt einen zwischen dem als Zugbolzen ausgebildeten Halteelement 9 und einem Ende der Öffnung 13 liegenden Bereich der Öffnung 13 im Wesentlichen vollständig aus. Die Einheit 14 ist im Bereich des Halteelementes 9 halbrund ausgebildet und umfasst somit das Halteelement 9 bzw. den Zugbolzen. Die Einheit 14 ist mit einem Haltemittel 29 lösbar fixiert. Das Haltemittel 29 bzw. der Haltebolzen durchsetzt den Block 12 und durchragt gemäß der Darstellung in Fig. 1 seitliche Ausnehmungen 17 des Blockes 12 normal zur Knochenplatte 5. In den seitlich vorragenden Teil des Haltemittels 29 greift ein Stellelement 15, nämlich eine Stellschraube ein. Das als Stellschraube ausgebildete Stellelement 15 durchsetzt den Block 12 parallel zur Verschieberichtung bzw. der Knochenplatte 5 und liegt auf Höhe des Haltemittels 29. Ist die Vorrichtung 1 in der in Fig. 1 dargestellten Form auf einer Ulna befestigt und bereits ein Knochenstück entnommen, so können zwei voneinander getrennte und beabstandete Teile der Ulna durch Drehen der Stellschraube zueinander bewegt werden, bis diese aneinander anliegen. Da sich bei drehender Stellschraube die Einheit 14 nicht bewegt, muss sich der übrige Teil des Blockes 12 mit der verbundenen Knochenplatte 5 in der in Fig. 1 durch einen Pfeil angezeigten Verschieberichtung bewegen, wobei das Halteelement 9 einen freien Bereich 23 der Öffnung 13 zumindest teilweise durchläuft. Der übrige Teil des Blockes 12 läuft dabei über das Haltemittel 29.

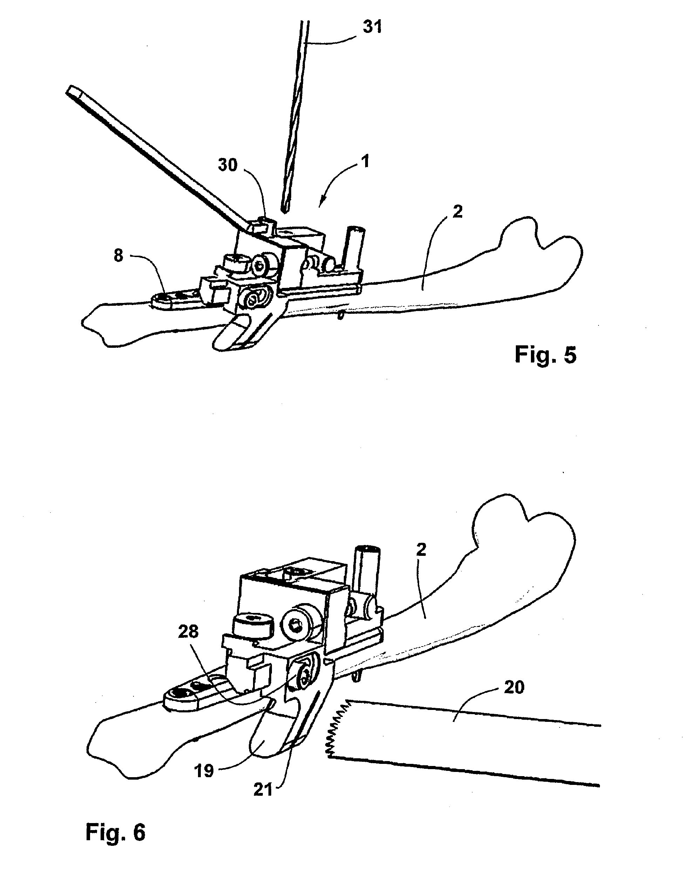

Anhand der Fig. 5 bis 8 ist die Verwendung der Vorrichtung 1 gemäß Fig. 1 bis 4 bei einer Operation bzw. einer Verkürzung einer Ulna schematisch dargestellt. Die Vorrichtung 1 wird auf einen Knochen 2 wie eine Ulna aufgesetzt, nachdem der Knochen 2 für die Operation freigelegt worden ist. Nach Aufsetzen der Vorrichtung 1 wird diese endseitig am Loch 6 mit einem Befestigungselement 8 wie einer Schraube am Knochen 2 fixiert. Des Weiteren werden in der Öffnung 13 des Blockes 12 bzw. im Langloch 7 der Knochenplatte 5 und den endseitigen Langlöchern 11, 16 die fußseitig ein Gewinde aufweisenden Halteelemente 9, 18 angeordnet und im Knochen 2 befestigt. Hierfür wird, wie aus Fig. 5 ersichtlich ist, in den freien Bereich 23 der Öffnung 13 ein passender Adapter 30 eingeführt. Dieser Adapter 30 ist passgenau für den freien Bereich 23 bzw. die Langlöcher 7, 11, 16 ausgebildet und weist eine vertikal verlaufende Öffnung auf, durch welche ein Bohrer 31 geführt werden kann. Aufgrund einer dimensionsgleichen Ausbildung des freien Bereichs 23 und der Langlöcher 7, 11, 16 ist der Adapter 30 mehrfach einsetzbar und dient sowohl beim Bohren eines Lochs im Knochen 2 für das Halteelement 9 als auch das Halteelement 18 als Führungs- und Positionierhilfe.

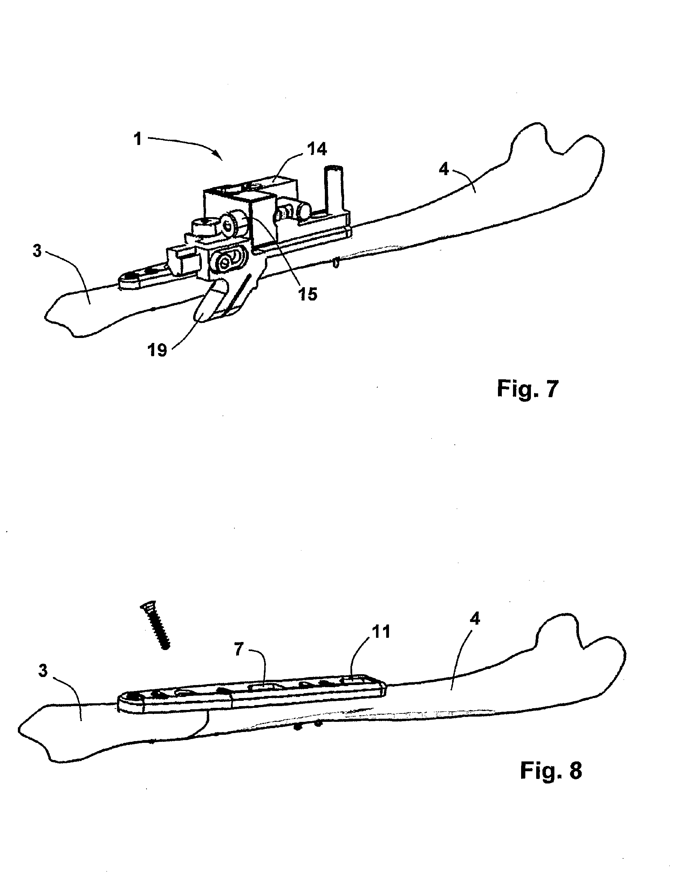

Ist die Vorrichtung 1 am Knochen 2 befestigt, so kann mit einem geeigneten Schneidgerät 20 eine erste Durchtrennung des Knochens 2 vorgenommen werden (Fig. 6). Nach erfolgtem ersten Durchtrennen des Knochens 2 wird das Halteelement 28 gelöst und die Führungseinrichtung 19 entsprechend einer Länge eines zu entnehmenden Knochenstücks auf der Schiene 27 verschoben und anschließend mit dem Halteelement 28 wieder fixiert, ehe ein zweiter Schnitt vorgenommen wird. Im Anschluss an den zweiten Schnitt wird das Knochenstück vorbestimmter Länge entnommen. Es ist nun eine wie in Fig. 7 dargestellte Situation erreicht, wobei Knochenteile 3, 4 noch voneinander beabstandet sind. Die durch Entnahme des Knochenstücks entstandene Lücke ist in Fig. 7 durch die Führungseinrichtung 19 der Vorrichtung 1 verdeckt. Mithilfe der Stellschraube bzw. des Stellelementes 15 wird nun der Knochenteil 3 in der zuvor beschriebenen Art zum Knochenteil 4 bewegt. Sobald der Knochenteil 3 am Knochenteil 4 anliegt, kann die Knochenplatte 5 auch am Knochenteil 4 fixiert werden. Wenn die Knochenplatte 5 mit Schrauben an beiden Knochenteilen 3, 4 fixiert ist, kann die Vorrichtung 1 abgenommen werden. Es ist dann eine in Fig. 8 dargestellte Situation erreicht. Bei Bedarf kann noch eine weitere Schraube in den Knochen getrieben werden, und zwar schräg im Bereich der Verbindungsflächen der Knochenteile 3, 4, sodass die Knochenteile 3, 4 zusätzlich aneinandergepresst gehalten werden.

## Patentansprüche

1. Vorrichtung (1) zum Verkürzen eines länglichen Knochens (2), insbesondere einer Ulna, umfassend ein Halteelement (9) und eine am Knochen (2) anzubringende Knochenplatte (5) mit einem Loch (6) für ein Befestigungselement (8) zum Fixieren der Knochenplatte (5) am Knochen (2) und einem längs dazu versetzten Langloch (7) für das Halteelement (9), das im Knochen (2) befestigbar ist, sodass Knochenteile (3, 4) nach Durchtrennen des Knochens (2) und Entnahme eines Knochenstücks im Bereich zwischen dem Loch (6) und dem Langloch (7) durch Verschieben der Knochenplatte (5) relativ zum Halteelement (9) zusammenführbar sind, **dadurch gekennzeichnet, dass** auf der Knochenplatte (5) im Bereich des Langlochs (7) ein Block (12) mit einer in Draufsicht länglichen und über dem Langloch (7) liegenden Öffnung (13) für das Halteelement (9) angeordnet ist, wobei in der Öffnung (13) eine Einheit (14) des Blockes (12) angeordnet ist, an welcher das Halteelement (9) in Verschieberichtung zur Anlage kommt und relativ zu welcher der übrige Teil des Blockes (12) mithilfe eines vorgesehenen Stellelementes (15) verschiebbar ist.

2. Vorrichtung (1) nach Anspruch 1, **dadurch gekennzeichnet, dass** die Knochenplatte (5) länglich ausgebildet ist.

3. Vorrichtung (1) nach Anspruch 1 oder 2, **dadurch gekennzeichnet, dass** der Block (12) bodenseitig mit Fortsätzen ausgebildet ist, die eine weitere Platte (10) definieren, die zumindest teilweise etwa mit einer gleichen Breite wie die Knochenplatte (5), jedoch kürzer ausgebildet ist.

4. Vorrichtung (1) nach Anspruch 3, **dadurch gekennzeichnet, dass** die weitere Platte (10) ein Langloch (16) und die Knochenplatte (5) ein weiteres Langloch (11) aufweist, welche Langlöcher (11, 16) übereinander angeordnet sind und sich an einem dem Loch (6) gegenüberliegenden Ende der Knochenplatte (5) befinden.

5. Vorrichtung (1) nach Anspruch 4, **dadurch gekennzeichnet, dass** das Halteelement (9) und ein weiteres Halteelement (18) vorgesehen sind, wobei das Halteelement (9) in das Langloch (7) der Knochenplatte (5) und die Öffnung (13) des Blockes (12) und das weitere Halteelement (18) in das weitere Langloch (11) der Knochenplatte (5) und in das Langloch (16) der weiteren Platte (10) eingreifen und länglich mit einem zylindrischen Teil und einem ein Gewinde aufweisenden Teil gebildet sind, wobei der zylindrische Teil des Halteelementes (9) im Block (12) an der Einheit (14) und der zylindrische Teil des weiteren Halteelementes (18) auf der weiteren Platte (10) anliegt.

6. Vorrichtung (1) nach Anspruch 5, **dadurch gekennzeichnet, dass** die Langlöcher (7, 11) der Knochenplatte (5) und ein freier Bereich der Öffnung (23), in den das Halteelement (9) eingeführt ist, und das Langloch (16) der weiteren Platte (10) mit etwa gleichen Abmessungen ausgebildet sind.

7. Vorrichtung (1) nach einem der Ansprüche 3 bis 6, **dadurch gekennzeichnet, dass** die weitere Platte (10) lösbar auf der Knochenplatte (5) angebracht ist.

8. Vorrichtung (1) nach einem der Ansprüche 3 bis 7, **dadurch gekennzeichnet, dass** an der weiteren Platte (10) eine Führungseinrichtung (19) für ein Schneidgerät (20) lösbar befestigt ist.

9. Vorrichtung (1) nach Anspruch 8, **dadurch gekennzeichnet, dass** die Führungseinrichtung (19) relativ zur weiteren Platte (10) variabel fixierbar ist.

10. Vorrichtung (1) nach Anspruch 8 oder 9, **dadurch gekennzeichnet, dass** die Führungseinrichtung (19) einen Führungsschlitz (21) und eine Skala (22) aufweist, an welcher eine Position der Führungseinrichtung (19) relativ zur weiteren Platte (10) ablesbar ist.

11. Vorrichtung (1) nach einem der Ansprüche 8 bis 10, **dadurch gekennzeichnet, dass** die Führungseinrichtung (19) lösbar auf der weiteren Platte (10) befestigt ist.

12. Vorrichtung (1) nach einem der Ansprüche 1 bis 11, **dadurch gekennzeichnet, dass** der übrige Teil des Blockes (12) durch das Stellelement (15) kontinuierlich verschiebbar ist.

13. Vorrichtung (1) nach einem der Ansprüche 1 bis 12, **dadurch gekennzeichnet, dass** die Einheit (14) das Halteelement (9) teilweise umfasst.

14. Vorrichtung (1) nach einem der Ansprüche 1 bis 13, **dadurch gekennzeichnet, dass** das Stellelement (15) als Stellschraube ausgebildet ist, welche im oder am übrigen Teil des Blockes (12) angeordnet ist und mit der Einheit (14) in Verbindung steht.

15. Vorrichtung (1) nach Anspruch 14, **dadurch gekennzeichnet, dass** die Stellschraube etwa parallel zur Knochenplatte (5) in Verschieberichtung verläuft.

16. Vorrichtung (1) nach einem der Ansprüche 1 bis 15, **dadurch gekennzeichnet, dass** die Einheit (14) in die Öffnung (13) einsetzbar ist und der Block (12) in Verschieberichtung verlaufende seitliche Ausnehmungen (17) aufweist, durch welche ein mit dem Stellelement (15) in Verbindung stehendes und die Einheit (14) vertikal in Position haltendes Haltemittel (29) geführt ist.

## Claims

1. Device (1) for shortening a long bone (2), more particularly an ulna, comprising a holding element (9) and, to be applied to the bone (2), a bone plate (5) with a hole (6) for a fastening element (8) for fixing the bone plate (5) to the bone (2) and an elongated hole (7), offset longitudinally thereto, for the holding element (9) that can be fastened in the bone (2), so that after severing the bone (2) and removing a section of bone, bone parts (3, 4) can be brought together in the area between the hole (6) and the elongated hole (7) by displacement of the bone plate (5) relative to the holding element (9), **characterised in that** arranged on the bone plate (5) in the area of the elongated hole (7) is a block (12) with, when viewed from above, an opening (13), that is elongated and lies above the elongated hole (7), for the holding element (9), wherein arranged in the opening (13) there is a unit (14) of the block (12) onto which the holding element (9) comes into contact in the direction of displacement and relative to which the remaining part of the block (12) can be displaced with the aid of provided adjusting element (15).

2. Device (1) according to claim 1 **characterised in** the bone plate (5) is elongated in design.

3. Device (1) according to claim 1 or 2 **characterised in that** the block (12) has projections on the base which define a further plate (10) which is at least partially the same width as the bone plate (5), but is designed to be shorter.

4. Device (1) according to claim 3 **characterised in that** the further plate (10) has an elongated hole (16) and the bone plate (5) has a further elongated hole (11), said elongated holes (11, 16) being arranged one on top of the other and located on an end of the bone plate (5) which is opposite the hole (6).

5. Device (1) according to claim 4 **characterised in that** the holding element (9) and a further holding element (18) are provided, wherein the holding element (9) engages in the elongated hole (7) of the bone plate (5) and the opening (13) of the block (12) and the further holding element (18) engages in the further elongated hole (11) of the bone plate (5) and into the elongated hole (16) of the further plate (10) and are designed longitudinally with a cylindrical section and a section with a thread, wherein the cylindrical part of the holding element (9) is in contact in the block (12) on the unit (14) and the cylindrical part of the further holding element (18) on the further plate (10) .

6. Device (1) according to claim 5 **characterised in that** the longitudinal holes (7, 11) of the bone plate (5) and a free area of the opening (23), in which the holding element (9) is inserted, and the elongated hole (16) of the further plate (10) have approximately the same dimensions.

7. Device (1) according to any one of claims 3 to 6 **characterised in that** the further plate (10) is applied to the bone plate (5) in a detachable manner.

8. Device (1) according to any one of claims 3 to 7 **characterised in that** guiding element (19) for a cutting device (20) is fastened on the further plate (10) in a detachable manner.

9. Device (1) according to claim 8 **characterised in that** the guiding element (19) is variably fixable relative to the further plate (10).

10. Device (1) according to claim 8 or 9 **characterised in that** the guiding element (19) has a guide slot (21) and a scale (22) on which the position of the guiding element (19) relative to the further plate (10) can be read off.

11. Device (1) according to any one of claims 1 to 10 **characterised in that** the guiding element (19) is attached to the further plate (10) in a detachable manner.

12. Device (1) according to any one of claims 1 to 11 **characterised in that** the remaining part of the block (12) can be continuously displaced by the adjusting element (15).

13. Device (1) according to any one of claims 1 to 12 **characterised in that** the unit (14) partially surrounds the holding element (9).

14. Device (1) according to any one of claims 1 to 13 **characterised in that** the adjusting element (15) is designed as an adjusting screw which is arranged in or on the remaining part of the block (12) and is in connection with the unit (14).

15. Device (1) according to claim 14 **characterised in that** the adjusting screw runs approximately in parallel with the bone plate (5) in the direction of displacement.

16. Device (1) according to any one of claims 1 to 15 **characterised in that** the unit (14) can be inserted into the opening (13) and the block (12) has lateral recesses (17) running in the direction of displacement through which a holding element (29), connected to the adjusting element (15) and holding the unit (14) vertically in position, is guided.

## Revendications

1. Dispositif (1) de raccourcissement d'un os oblong (2), en particulier d'un cubitus, comprenant un élément de retenue (9) et une plaque osseuse (5) à fixer à l'os (2) et percée d'un trou (6) pour un élément de fixation (8) servant à fixer la plaque osseuse (5) à l'os (2) et un trou oblong (7) décalé longitudinalement pour l'élément de retenue (9) qui peut être fixé dans l'os (2), de sorte que les parties osseuses (3, 4), après sectionnement de l'os (2) et prélèvement d'un morceau d'os dans la partie située entre le trou (6) et le trou oblong (7), peuvent être réunies en décalant la plaque osseuse (5) par rapport à l'élément de retenue (9), **caractérisé en ce que**, sur la plaque osseuse (5), au niveau du trou oblong (7), est disposé un bloc (12) doté d'une ouverture (13) allongée vue du dessus et située au-dessus du trou oblong (7) pour l'élément de retenue (9), étant disposée dans l'ouverture (13) une unité (14) du bloc (12) sur laquelle l'élément de retenue (9) vient se poser dans le sens de déplacement et par rapport à laquelle le reste du bloc (12) est déplaçable à l'aide d'un élément de positionnement (15) prévu.

2. Dispositif (1) selon la revendication 1, **caractérisé en ce que** la plaque osseuse a une conformation oblongue.

3. Dispositif (1) selon la revendication 1 ou 2, **caractérisé en ce que** le bloc (12) est réalisé au niveau du fond avec des prolongements qui définissent une autre plaque (10) qui est réalisée du moins partiellement approximativement en même largeur que la plaque osseuse (5) mais est plus courte.

4. Dispositif (1) selon la revendication 3, **caractérisé en ce que** l'autre plaque (10) présente un trou oblong (16) et la plaque osseuse (5) un autre trou oblong (11), lesquels trous oblongs (11, 16) sont superposés et se trouvent à une extrémité opposée au trou (6) de la plaque osseuse (5).

5. Dispositif (1) selon la revendication 4, **caractérisé en ce que** sont prévus l'élément de retenue (9) et un autre élément de retenue (18), l'élément de retenue (9) s'engrenant dans le trou oblong (7) de la plaque osseuse (5) et l'ouverture (13) du bloc (12) et l'autre élément de retenue (18) s'engrenant dans l'autre trou oblong (11) de la plaque osseuse (5) et dans le trou oblong (16) de l'autre plaque (10) et étant réalisés longitudinalement avec une partie cylindrique et une partie présentant un filetage, la partie cylindrique de l'élément de retenue (9) reposant dans le bloc (12) sur l'unité (14) et la partie cylindrique de l'autre élément de retenue (18) sur l'autre plaque (10).

6. Dispositif (1) selon la revendication 5, **caractérisé en ce que** les trous oblongs (7, 11) de la plaque osseuse (5) et une zone dégagée de l'ouverture (23) dans laquelle l'élément de retenue (9) est introduit et le trou oblong (16) de l'autre plaque (10) sont réalisés à peu près en mêmes dimensions.

7. Dispositif (1) selon une des revendications 3 à 6, **caractérisé en ce que** l'autre plaque (10) est installée de manière amovible sur la plaque osseuse (5) .

8. Dispositif (1) selon une des revendications 3 à 7, **caractérisé en ce qu'**un dispositif de guidage (19) pour un appareil de coupe (20) peut être fixé de manière amovible sur l'autre plaque (10).

9. Dispositif (1) selon la revendication 8, **caractérisé en ce que** le dispositif de guidage (19) peut être fixé de manière variable par rapport à l'autre plaque (10).

10. Dispositif (1) selon la revendication 8 ou 9, **caractérisé en ce que** le dispositif de guidage (19) présente une fente de guidage (21) et une graduation (22) sur laquelle une position du dispositif de guidage (19) par rapport à l'autre plaque (10) peut être consultée.

11. Dispositif (1) selon une des revendications 8 à 10, **caractérisé en ce que** le dispositif de guidage (19) peut être fixé de manière amovible sur l'autre plaque (10) .

12. Dispositif (1) selon une des revendications 1 à 11, **caractérisé en ce que** le reste du bloc (12) peut être déplacé en continu par l'élément de positionnement (15) .

13. Dispositif (1) selon une des revendications 1 à 12, **caractérisé en ce que** l'unité (14) entoure partiellement l'élément de retenue (9).

14. Dispositif (1) selon une des revendications 1 à 13, **caractérisé en ce que** l'élément de positionnement (15) se présente sous forme d'une vis de réglage qui est disposée dans ou sur le reste du bloc (12) et est en liaison avec l'unité (14).

15. Dispositif (1) selon la revendication 14, **caractérisé en ce que** la vis de réglage s'étend à peu près parallèlement à la plaque osseuse (5) dans le sens de déplacement.

16. Dispositif (1) selon une des revendications 1 à 15, **caractérisé en ce que** l'unité (14) peut être insérée dans l'ouverture (13) et le bloc (12) présente des échancrures latérales (17) s'étendant dans le sens de déplacement et dans lesquelles passe un moyen de retenue (29) en liaison avec l'élément de positionnement (15) et maintenant verticalement l'unité (14) en position.
